(19) **Europäisches Patentamt / European Patent Office / Office européen des brevets**

(11) **EP 4 174 109 A1**

(12) # EUROPEAN PATENT APPLICATION
published in accordance with Art. 153(4) EPC

(43) Date of publication:
**03.05.2023 Bulletin 2023/18**

(21) Application number: **22789828.5**

(22) Date of filing: **11.07.2022**

(51) International Patent Classification (IPC):
**C08G 65/06** $^{(2006.01)}$

(86) International application number:
**PCT/JP2022/027315**

(87) International publication number:
**WO 2023/042533 (23.03.2023 Gazette 2023/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2021 JP 2021150536**

(71) Applicant: **Sumitomo Seika Chemicals Co., Ltd.**
**Kako-gun, Hyogo 675-0145 (JP)**

(72) Inventors:
• **IMAMURA, Yuika**
**Himeji-shi**
**Hyogo**
**6728076 (JP)**
• **KIMURA, Akira**
**Himeji-shi**
**Hyogo**
**6728076 (JP)**

(74) Representative: **Vossius & Partner**
**Patentanwälte Rechtsanwälte mbB**
**Siebertstraße 3**
**81675 München (DE)**

(54) **POLYALKYLENE OXIDE, SOLID PREPARATION-USE COMPOSITION AND METHOD FOR MANUFACTURING SAME, AND CONTROLLED RELEASE PREPARATION-USE COMPOSITION**

(57)     Provided is a polyalkylene oxide that can be suitably used to easily control the drug dissolution rate, a solid dosage form composition comprising the polyalkylene oxide and a method for producing the same, and a sustained-release dosage form composition.

The polyalkylene oxide of the present invention has a polydispersity of 3 to 5, a gel strength of 22500 Pa or more, and an erosion of 40 to 48%. The solid dosage form composition of the present invention comprises at least the polyalkylene oxide, an active component, and an excipient.

**Description**

Technical Field

The present invention relates to a polyalkylene oxide, a solid dosage form composition comprising the polyalkylene oxide and a method for producing the same, and a sustained-release dosage form composition.

Background Art

Solid preparations are widely used for pharmaceutical applications. In particular, sustained-release solid preparations are indispensable for pharmaceutical applications because the blood levels of active ingredients can be kept in a constant range for a long period of time by controlling the dissolution rate of the active ingredients from the solid dosage forms.

PTL 1 and others disclose techniques for obtaining solid dosage forms with controlled sustained release by using specific modified starch and a dissolution control agent. In recent years, development has proceeded from various viewpoints to control the dissolution rate of solid dosage forms, as exemplified by such techniques.

Citation List

Patent Literature

PTL 1: JP2014-9208A

Summary of Invention

Technical Problem

In general, when the dissolution rate of active ingredients is controlled in solid dosage forms, the type and amount of drugs contained in solid dosage forms are fixed in the preparations, and the dissolution rate thus cannot be controlled by changing the type and amount of the drugs.

It is also possible to control the dissolution rate of active ingredients by changing the shape and size of solid dosage forms; however, in this case, it is necessary to change the design etc. of the equipment used to obtain solid dosage forms, which is not realistic from the viewpoint of cost etc. It is also possible to finely control the dissolution rate by changing the type and amount of excipients contained in preparations; however, in this case, it is necessary to clean the production equipment due to changes in substances and blending amounts. Furthermore, it is also necessary to conduct dissolution rate confirmation tests multiple times, which takes an enormous amount of time and money to establish an optimal production process. From the above points of view, it has been desired to establish a technique that can easily control the dissolution rate of active ingredients without changing the preparation process.

The present invention was made in view of the above, and its object is to provide a polyalkylene oxide that can be suitably used to easily control the drug dissolution rate, a solid dosage form composition comprising the polyalkylene oxide and a method for producing the same, and a sustained-release dosage form composition.

Solution to Problem

The present inventors conducted extensive research to achieve the above object, and consequently found that the above object can be achieved by setting the polydispersity, gel strength, and erosion of the polyalkylene oxide within predetermined ranges. Thus, the present invention has been completed.

Specifically, the present invention includes, for example, the main subjects described in the following items.

Item 1.

A polyalkylene oxide having a polydispersity of 3 to 5, a gel strength of 22500 Pa or more, and an erosion of 40 to 48%.

Item 2.

A solid dosage form composition comprising at least the polyalkylene oxide according to Item 1, an active component, and an excipient.

Item 3.

A sustained-release dosage form composition comprising at least the polyalkylene oxide according to Item 1, an active component, and an excipient.

Item 4.

A method for producing a solid dosage form composition, comprising:

dry-mixing the polyalkylene oxide according to Item 1 and an active component to obtain a mixture; and tableting the mixture to obtain tablets.

Item 5.

A method for producing a solid dosage form composition, comprising:

obtaining granules comprising the polyalkylene oxide according to Item 1 and an active component; and tableting the granules to obtain tablets.

Item 6

Use of the polyalkylene oxide according to Item 1 in a sustained-release dosage form.

Advantageous Effects of Invention

The polyalkylene oxide according to the present invention can be suitably used as a solid dosage form raw material for easily controlling the drug dissolution rate.
The solid dosage form composition according to the present invention comprises the above polyalkylene oxide, and thus can form a solid dosage form that can easily control the drug dissolution rate.

Description of Embodiments

Embodiments of the present invention are described in detail below. In the present specification, the terms "comprising" and "containing" include the concepts of "comprising," "containing," "consisting essentially of," and "consisting of."
In the numerical range described in stages in the present specification, the upper or lower limit of the numerical range at one stage can be optionally combined with the upper or lower limit of the numerical range at another stage. In the numerical range described in the present specification, the upper or lower limit of the numerical range may be replaced with a value shown in the Examples or a value that can be uniquely derived from the Examples. Further, in the present specification, the numerical values connected by "to" mean the numerical range including the numerical values before and after "to" as the lower limit and the upper limit.

1. Polyalkylene Oxide

The polyalkylene oxide of the present invention has a polydispersity of 3 to 5, a gel strength of 22500 Pa or more, and an erosion of 40 to 48%. This polyalkylene oxide can be suitably used as a solid dosage form raw material (particularly a sustained-release dosage form raw material) for easily controlling the drug dissolution rate. In particular, the dissolution rate can be controlled lower.
In the polyalkylene oxide, the number of carbon atoms in the alkylene moiety is not particularly limited, and is preferably 2 or more and 4 or less, for example. Because the effects of the present invention can be easily exhibited, it is particularly preferable that the alkylene moiety has 2 carbon atoms; that is, the polyalkylene oxide is preferably polyethylene oxide.
The mass average molecular weight of the polyalkylene oxide is not particularly limited, and is, for example, 200,000 or more, preferably 500,000 or more, more preferably 1 million or more, even more preferably 1.2 million or more, and particularly preferably 1.5 million or more. The mass average molecular weight of the polyalkylene oxide as mentioned herein refers to a value measured by gel permeation chromatography, and particularly refers to a value calculated from a calibration curve created using a known polyethylene oxide standard sample.
The polydispersity, i.e., mass average molecular weight (Mw)/number average molecular weight (Mn), of the polyalkylene oxide is 3 to 5 (3 or more and 5 or less), as described above. If the polydispersity is less than 3 or more than 5, the dissolution rate cannot be controlled within a desired range. That is, because the polydispersity of the polyalkylene oxide is 3 to 5, the erosion (dissolution rate) of the polyalkylene oxide can be easily controlled within a desired range, and the dissolution rate can be further reduced. The polydispersity of the polyalkylene oxide is preferably 3.1 or more, and preferably 4.5 or less.

The methods for adjusting the mass average molecular weight and polydispersity of the polyalkylene oxide are not particularly limited, and known methods can be widely used. For example, these can be adjusted according to the conditions of the polymerization reaction in the production of the polyalkylene oxide. Specifically, the mass average molecular weight and polydispersity of the polyalkylene oxide can be adjusted by adjusting the type and amount of raw material used in the polymerization reaction. Further, the mass average molecular weight and polydispersity of the polyalkylene oxide can be adjusted by using a chain transfer agent, described later, in the polymerization reaction. In a particularly preferred method, the mass average molecular weight and polydispersity of the polyalkylene oxide are adjusted by irradiating a polyalkylene oxide, whose molecular weight is adjusted in advance to be higher than the target molecular weight, with active energy rays, such as gamma rays, as described later.

The gel strength of the polyalkylene oxide is 22500 Pa or more, as described above. If the gel strength is less than 22500 Pa, the gel strength becomes low, and the dissolution rate becomes high; the elution rate cannot be controlled within a desired range. That is, because the gel strength of the polyalkylene oxide is 22500 Pa or more, the erosion (dissolution rate) of the polyalkylene oxide can be easily controlled within a desired range, and the dissolution rate can be further reduced. The gel strength of the polyalkylene oxide is preferably 23000 Pa or more, and more preferably 24000 Pa or more. The upper limit of the gel strength of the polyalkylene oxide is not particularly limited, and is 29000 Pa, for example.

In the present specification, the gel strength of the polyalkylene oxide refers to a value measured in the following manner. First, 200 mg of polyalkylene oxide is placed in a cylindrical mortar with a diameter of 10 mm and compressed with a compression force of 5 kN to obtain tablets. Using the tablets, a dissolution test is performed according to the Japanese Pharmacopoeia (paddle method). In the dissolution test, the test liquid is ion-exchange water, the test temperature is 37°C, and the stirring speed is 200 rpm. The dissolution test is performed by stirring under these conditions for 2.5 hours to gelate the tablets. Using the gelled tablets as measurement samples, a compression test is performed using a compact compression/tensile tester (EZ-Test, produced by Shimadzu Corporation). In the compression test, an indenter with a diameter of 10 mm is used, and the displacement rate is 5 mm/min. Based on a stress-strain curve obtained by the compression test, the slope S (Pa) when the gelled tablets are displaced from 5% to 10% is derived. The above series of operations are performed three times in total with different tablets, and the slope S (Pa) of each case is derived. The average value of these is taken as the gel strength of the polyalkylene oxide.

The method for adjusting the gel strength of the polyalkylene oxide is not particularly limited, and known methods can be widely used. For example, the mass average molecular weight and polydispersity of the polyalkylene oxide can be adjusted to thereby achieve a desired gel strength.

The erosion (dissolution rate) of the polyalkylene oxide is 40 to 48% (40% or more and 48% or less), as described above. Because the erosion of the polyalkylene oxide is 40 to 48%, the dissolution rate of the polyalkylene oxide can be controlled low. If the erosion of the polyalkylene oxide is lower than 40%, the dissolution rate is too low, which is not suitable for solid dosage form. An erosion of the polyalkylene oxide exceeding 48% is not suitable for solid dosage form for which a low dissolution rate is required. The erosion of the polyalkylene oxide is preferably 43% or more, and preferably 48% or less.

In the present specification, the erosion (dissolution rate) of the polyalkylene oxide refers to a value measured in the following manner. First, 200 mg of polyalkylene oxide is placed in a cylindrical mortar with a diameter of 10 mm and compressed with a compression force of 5 kN to obtain tablets. Using the tablets, a dissolution test is performed according to the Japanese Pharmacopoeia (paddle method). In the dissolution test, the test liquid is ion-exchange water, the test temperature is 37°C, and the stirring speed is 200 rpm. The dissolution test is performed by stirring under these conditions for 4.5 hours to gelate the tablets. The gelled tablets are taken out and dried at 80°C for 5 hours, and the erosion (dissolution rate) is calculated from the following formula (1):

$$\text{Erosion (\%)} = \{1-(A/B)\} \times 100 \quad (1)$$

In formula (1), A represents tablet mass after drying performed after the dissolution test, and B represents tablet mass before the dissolution test.

The erosion of the polyalkylene oxide can be adjusted within a desired range, for example, by adjusting the mass average molecular weight and polydispersity of the polyalkylene oxide.

The viscosity (2% aqueous solution viscosity) of the polyethylene oxide is not particularly limited. For example, the lower limit is 50 mPa/s or more, preferably 1000 mPa/s or more, more preferably 1500 mPa/s or more, and particularly preferably 2000 mPa/s or more. The upper limit is 6000 mPa/s or less, preferably 5000 mPa/s or less, more preferably 4500 mPa/s or less, and particularly preferably 4000 mPa/s or less. The 2% aqueous solution viscosity can be measured using a rotational viscometer (RV DVII+, produced by Brookfield).

The polyalkylene oxide is generally a homopolymer, but is not limited thereto, and may be a copolymer. When the polyalkylene oxide is a copolymer, the polyalkylene oxide has, for example, two or more structural units with alkylene moieties having different number of carbon atoms.

The polyalkylene oxide of the present invention may be a single polyalkylene oxide or a mixture of two or more polyalkylene oxides, and may be, for example, a mixture of polyalkylene oxides produced by different production methods.

The method for producing the polyalkylene oxide is not particularly limited. For example, methods for producing known polyalkylene oxides can be widely used. Specifically, the polyalkylene oxide can be produced by polymerization reaction of an alkylene oxide in the presence of a catalyst.

Examples of the alkylene oxide used in the production of the polyalkylene oxide include aliphatic alkylene oxides. Specific examples include ethylene oxide, propylene oxide, and butylene oxide; preferably ethylene oxide or propylene oxide; and particularly preferably ethylene oxide.

The type of catalyst used in the production of the polyalkylene oxide is also not particularly limited. For example, catalysts used in methods for producing known polyalkylene oxides can be widely used. Specifically, metal catalysts such as zinc can be used. The amount of catalyst used is also not particularly limited, and can be set, for example, within a range similar to that of methods for producing known polyalkylene oxides.

A chain transfer agent can be used in the polymerization reaction of the alkylene oxide. This can easily adjust the molecular weight of the polyalkylene oxide. The type of chain transfer agent is not particularly limited, and known chain transfer agents that can be used in the polymerization reaction of alkylene oxides can be widely used. Examples of chain transfer agents include $C_{1-5}$ alcohol compounds, such as methanol, ethanol, n-propanol, isopropanol, n-butanol, isobutanol, and t-butanol.

When a chain transfer agent is used, the amount thereof used is not particularly limited, and can be, for example, 0.002 to 0.3 mol% based on the alkylene oxide.

A solvent can be used, if necessary, in the polymerization reaction of the alkylene oxide. For example, solvents used in methods for producing known polyalkylene oxides can be widely used.

The temperature and other conditions of the polymerization reaction of the alkylene oxide are also not particularly limited, and can be the same as known conditions.

The polyalkylene oxide obtained by the above polymerization reaction can be irradiated with active energy rays, if necessary. As a result, the polyalkylene oxide is decomposed, and the molecular weight can be adjusted within a desired range. Examples of active energy rays include radiation such as gamma rays, ultraviolet rays, X-rays, ion beam, and the like. In terms of the ease of adjusting the molecular weight, gamma rays are preferred.

The irradiation conditions of active energy rays are also not particularly limited, and can be suitably set, for example, so as to achieve the desired molecular weight and polydispersity. For example, 0.1 to 20 kGy of gamma rays can be applied depending on the type of polyalkylene oxide. When irradiation with gamma rays at a relatively low radiation dose is sufficient, for example, the radiation dose can be 0.3 to 1 kGy, and preferably 0.5 to 0.8 kGy.

The polyalkylene oxide of the present invention may be a mixture of two or more polyalkylene oxides, and may be, for example, a mixture of polyalkylene oxides each obtained by irradiation with gamma rays at different radiation doses. That is, the polyalkylene oxide of the present invention can be obtained by mixing a polyalkylene oxide obtained by irradiation with gamma rays at a radiation dose X1, and a polyalkylene oxide obtained by irradiation with gamma rays at a radiation dose X2, which is different from the radiation dose X1. For the physical properties, such as polydispersity, gel strength, and erosion, of the polyalkylene oxide mixture, values obtained by measuring the mixture are used.

## 2. Solid Dosage Form Composition

The solid dosage form composition of the present invention comprises at least the polyalkylene oxide, an active component, and an excipient. The solid dosage form composition of the present invention is suitable as a raw material for obtaining solid dosage forms. In particular, since the solid dosage form composition of the present invention comprises the polyalkylene oxide, solid dosage forms with a controlled dissolution rate of active components can be prepared, and it is particularly suitable to obtain solid dosage forms with a lower dissolution rate.

In the solid dosage form composition, the type of active component is not particularly limited, and known active components can be widely applied.

In the solid dosage form composition, as the excipient, pharmaceutically acceptable excipients can be widely applied. For example, known excipients can be widely applied.

In the solid dosage form composition, the content of the active component is not particularly limited, and can be set within an appropriate range depending on the dose, usage, etc. of the solid dosage form.

In the solid dosage form composition, the content of the excipient is not particularly limited, and can be set within an appropriate range depending on the dose, usage, etc. of the solid dosage form.

In the solid dosage form composition, the content ratio of the polyalkylene oxide, active component, and excipient is not particularly limited, and can be, for example, the same as known solid dosage form compositions.

The solid dosage form composition may consist of the polyalkylene oxide, the active component, and the excipient. Alternatively, the solid dosage form composition may contain various components, such as various additives used for solid dosage forms, in addition to the polyalkylene oxide, the active component, and the excipient. Examples of such additives include disintegrants, fluidizers, lubricants, fragrances, colorants, and the like.

The solid dosage form composition can be widely used as a raw material for producing various solid dosage forms, and is suitable, for example, as a raw material for producing sustained-release dosage forms, described later. Therefore, it is a preferable embodiment that the solid dosage form in the solid dosage form composition of the present invention is a sustained-release dosage form.

The method for preparing a solid dosage form from the solid dosage form composition is not particularly limited. For example, methods similar to methods for preparing known solid dosage forms can be used.

The type of the solid dosage form is not particularly limited. For example, capsules, tablets, granules, and other known dosage forms can be widely applied.

Method for Producing Solid Dosage Form Composition

The method for producing the solid dosage form composition is not particularly limited. For example, methods for preparing known solid dosage form compositions can be widely used. For example, when the solid dosage form composition is in the form of tablets, the solid dosage form composition can be produced by production method A or B described below.

Production method A comprises the following steps A1 and A2:

step A1: dry-mixing a polyalkylene oxide and an active component to obtain a mixture; and
step A2: tableting the mixture to obtain tablets.

In step A1, a polyalkylene oxide and an active component are dry-mixed. The polyalkylene oxide used in step A1 is the polyalkylene oxide of the present invention described above. In dry-mixing, an excipient and optionally used various additives can also be added and mixed together with the polyalkylene oxide and the active component.

In step A1, the dry-mixing method is not particularly limited. For example, a known dry-mixing means can be applied. The mixing conditions are also not particularly limited, and can be suitably set depending on the type and amount of raw material used.

In step A2, the mixture obtained in step A1 is tableted to obtain tablets. The tableting method is not particularly limited. For example, a known tableting method can be applied.

Production method B comprises the following steps B1 and B2:

step B1: obtaining granules comprising a polyalkylene oxide and an active component; and
step B2: tableting the granules to obtain tablets.

In step B1, granules comprising a polyalkylene oxide and an active component are obtained. The polyalkylene oxide used in step B1 is the polyalkylene oxide of the present invention described above. In the step of obtaining granules, an excipient and optionally used additives can also be used together with the polyalkylene oxide and the active component.

In step B1, the method for obtaining granules is not particularly limited. For example, known granulation methods can be widely applied.

In step B2, the granules obtained in step B1 are tableted to obtain tablets. The tableting method is not particularly limited. For example, a known tableting method can be applied.

3. Sustained-Release Dosage Form Composition

The sustained-release dosage form composition of the present invention comprises the solid dosage form composition. That is, the sustained-release dosage form composition of the present invention comprises at least the polyalkylene oxide, the active component, and the excipient. The sustained-release dosage form composition of the present invention is suitable as a raw material for obtaining sustained-release dosage forms. In particular, since the sustained-release dosage form composition of the present invention comprises the polyalkylene oxide, sustained-release dosage forms with a controlled dissolution rate of active components can be prepared, and it is suitable for obtaining sustained-release dosage forms with a lower dissolution rate.

The sustained-release dosage form composition may consist of the solid dosage form composition, or may contain other additives, if necessary, in addition to the solid dosage form composition.

The method for preparing a sustained-release dosage form from the sustained-release dosage form composition is not particularly limited. For example, methods similar to methods for preparing known solid dosage forms can be used.

The dosage form of the sustained-release dosage form is not particularly limited. For example, capsules, tablets, granules, and other known dosage forms can be widely applied.

Since the solid dosage form composition and sustained-release dosage form composition described above each comprise the polyalkylene oxide, the dissolution rate of active ingredients from solid dosage forms prepared from these compositions can be easily controlled. In particular, the dissolution rate can be controlled low. Therefore, for solid dosage forms obtained using the solid dosage form composition and the sustained-release dosage form composition, the number of doses can be reduced, constant blood levels of active ingredients can be maintained for a long period of time, and side effects can be easily suppressed.

Examples

The present invention is described in more detail below with reference to Examples; however, the present invention is not limited to these Examples.

Production Example 1: Production of Zinc Catalyst

After the inside of a 500-mL round-bottom flask with an inner diameter of 80 mm equipped with a cooling device, a dropping funnel, a nitrogen gas inlet tube, and an impeller with four paddle blades with a blade diameter of 53 mm (inclined at 45 degrees) as a stirrer was replaced with nitrogen, 87.1 g of n-hexane and 9.90 g of diethyl zinc (Et2Zn) were added into the flask. While maintaining the internal temperature of the flask at 20°C and stirring inside the flask at a tip end peripheral speed of 0.97 m/sec (stirring speed: 350 rpm), as the first stage, 11.03 g (0.240 mol) of ethyl alcohol (EtOH) was added dropwise at 0.2 g/min, and the reaction was performed to obtain a reaction liquid. Subsequently, as the second stage, a mixed liquid of 6.49 g (0.072 mol) of 1,4-butanediol (1,4-BDO) and 13.27 g (0.288 mol) of ethyl alcohol was added dropwise at 0.2 g/min to the reaction liquid cooled to 10°C. After the completion of dropping, the inside of the flask was heated to 30°C, and the reaction was further continued for 1 hour. Then, the temperature was raised to 50°C, and the reaction was continued for 1 hour. Thereafter, unreacted components were removed by distillation by heating the flask to 80°C. After distillation, the inside of the flask was allowed to cool to room temperature, and 52.4 g of n-hexane was added, followed by heating to 80°C to perform the second distillation. This operation was repeated one more time, and a total of three distillations were performed. After cooling, the resultant was diluted with 264 g of n-hexane to obtain 297 g of zinc catalyst with a zinc content of 1.8 mass%.

Production Example 2-1: Production of High-Molecular-Weight Polyalkylene Oxide

After the inside of a 1-L pressure-resistant reaction vessel with an inner diameter of 94 mm equipped with a dropping funnel, a nitrogen gas inlet tube, and an impeller with an anchor-type paddle blade with a blade diameter of 47 mm as a stirrer was sufficiently replaced with nitrogen, 340 g of n-hexane was placed in the pressure-resistant reaction vessel, and 0.975 g (zinc equivalent: 0.0004 mol) of the zinc catalyst obtained in Production Example 1 was uniformly dispersed in the n-hexane to obtain a dispersion. After 81 g (1.84 mol) of ethylene oxide was added to the dispersion, the vessel was sealed, and the polymerization reaction was carried out while maintaining the temperature at 40°C and stirring. The white product obtained from the polymerization reaction was separated by filtration and dried under reduced pressure at 40°C to obtain 81.0 g of high-molecular-weight polyethylene oxide.

Production Example 2-2: Production of High-Molecular-Weight Polyalkylene Oxide

After the inside of a 1-L pressure-resistant reaction vessel with an internal diameter of 94 mm equipped with a dropping funnel, a nitrogen gas inlet tube, and an impeller with an anchor-type paddle blade with a blade diameter of 47 mm as a stirrer was sufficiently replaced with nitrogen, 340 g of n-hexane was placed in the pressure-resistant reaction vessel, and 0.975 g (zinc equivalent: 0.0004 mol) of the zinc catalyst obtained in Production Example 1 was uniformly dispersed in the n-hexane to obtain a dispersion. After 0.088 g (0.0012 mol) of t-butanol and 81 g (1.84 mol) of ethylene oxide were added to the dispersion, the vessel was sealed, and the polymerization reaction was carried out while maintaining the temperature at 40°C and stirring. The white product obtained from the polymerization reaction was separated by filtration and dried under reduced pressure at 40°C to obtain 81.0 g of high-molecular-weight polyethylene oxide.

Example 1-1

40.0 g of the high-molecular-weight polyethylene oxide obtained in Production Example 2-1 was irradiated with 0.8 kGy of gamma rays to obtain 40.0 g of the target polyethylene oxide.

Example 1-2

20.0 g of polyethylene oxide obtained by irradiating 20.0 g of the high-molecular-weight polyethylene oxide obtained in Production Example 2-1 with 0.6 kGy of gamma rays, and 20 g of polyethylene oxide obtained by irradiating 20.0 g of the high-molecular-weight polyethylene oxide obtained in Production Example 2-1 with 0.8 kGy of gamma rays were mixed to obtain 40.0 g of the target polyethylene oxide.

Example 1-3

40.0 g of the target polyethylene oxide was obtained in the same manner as in Example 1-1, except that the irradiation was changed to irradiation with 0.6 kGy of gamma rays.

Example 2-1

40.0 g of the target polyethylene oxide was obtained in the same manner as in Example 1-3.

Example 2-2

20.0 g of polyethylene oxide obtained by irradiating 20.0 g of the high-molecular-weight polyethylene oxide obtained in Production Example 2-1 with 0.75 kGy of gamma rays, and 20.0 g of polyethylene oxide obtained by irradiating 20.0 g of the high-molecular-weight polyethylene oxide obtained in Production Example 2-1 with 0.8 kGy of gamma rays were mixed to obtain 40.0 g of the target polyethylene oxide.

Example 2-3

40.0 g of the target polyethylene oxide was obtained in the same manner as in Example 1-1, except that the irradiation was changed to irradiation with 0.5 kGy of gamma rays.

Example 2-4

40.0 g of the target polyethylene oxide was obtained in the same manner as in Example 2-3.

Comparative Example 1-1

40.0 g of the high-molecular-weight polyethylene oxide obtained in Production Example 2-2 was irradiated with 0.3 kGy of gamma rays to obtain 40.0 g of the target polyethylene oxide.

Comparative Example 1-2

40.0 g of the target polyethylene oxide was obtained in the same manner as in Comparative Example 1-1.

Comparative Example 2-1

40.0 g of the target polyethylene oxide was obtained in the same manner as in Comparative Example 1-1, except that the irradiation was changed to irradiation with 0.2 kGy of gamma rays.

Comparative Example 2-2

40.0 g of the target polyethylene oxide was obtained in the same manner as in Comparative Example 2-1.

Table 1

| Example /Comparative Example | PEO physical properties | | | | PEO tablets | |
|---|---|---|---|---|---|---|
| | Mw | Mn | Mw/Mn (polydispersity) | 2% aqueous solution viscosity [mPa·s] | Gel strength [Pa] | Erosion [%] |
| Example 1-1 | 2083087 | 608544 | 3.4 | 2270 | 24414 | 47.8 |
| Example 1-2 | 1878407 | 519064 | 3.6 | 2630 | 25483 | 45.4 |
| Example 1-3 | 2025654 | 446981 | 4.5 | 2600 | 24423 | 46.3 |
| Comparative Example 1-1 | 1402989 | 195230 | 7.2 | 2500 | 18864 | 51.6 |
| Comparative Example 1-2 | 1590109 | 209543 | 7.6 | 2710 | 18714 | 51.9 |
| Example 2-1 | 2074076 | 540329 | 3.8 | 3620 | 28515 | 43.0 |
| Example 2-2 | 1936620 | 624014 | 3.1 | 3950 | 26903 | 43.1 |
| Example 2-3 | 2210683 | 516138 | 4.3 | 3610 | 24236 | 44.2 |
| Example 2-4 | 1789904 | 431338 | 4.1 | 3980 | 26443 | 43.1 |
| Comparative Example 2-1 | 1802905 | 257664 | 7.0 | 3700 | 18973 | 51.8 |
| Comparative Example 2-2 | 1586912 | 192272 | 8.3 | 3940 | 18449 | 48.2 |

Table 1 shows the mass average molecular weight (Mw), number average molecular weight (Mn), polydispersity, 2% aqueous solution viscosity, gel strength, and erosion (dissolution rate) of the polyethylene oxides obtained in the Examples and Comparative Examples.

From the results of Table 1, the obtained polyalkylene oxides had a polydispersity of 3 to 5, a gel strength of 22500 Pa or more, and an erosion of 40 to 48%. Therefore, it was revealed that when the polyalkylene oxides obtained in the Examples are used for preparation application, the dissolution rate of the drug (active component) can be controlled low.

The various evaluations methods used were as follows.

Mass Average Molecular Weight and Polydispersity

The mass average molecular weight and polydispersity of each polyethylene oxide were measured by gel permeation chromatography. Specifically, 0.02 g of polyethylene oxide was added and dissolved in 40 mL of a 0.19 M sodium nitrate aqueous solution over 3 hour, the resulting solution was filtered through a 0.8-pm membrane filter, and the obtained filtrate was subjected to measurement by gel permeation chromatography (HLC-8220GPC, produced by Tosoh Corporation; guardcolumn: TSKgel guardcolumn PWXL). In this measurement, the size exclusion columns were TSKgel G6000PWXL, TSKgel GMPWXL, and TSKgel G3000PWXL, the mobile phase was a 0.20 M sodium nitrate aqueous solution, the flow rate was 0.5 mL/min, the column temperature was 40°C, the differential refractometer temperature was 40°C, the injection volume was 500 $\mu$L, and the measurement time was 90 minutes. Separately from this, the same measurement was carried out using a polyethylene oxide standard sample with known mass average molecular weight, number average molecular weight, and polydispersity to create a calibration curve. Based on this calibration curve, the mass average molecular weight, number average molecular weight, and polydispersity of polyethylene oxides with a LogM of 3.5 to 7.2 were calculated.

2% Aqueous Solution Viscosity

12 g of polyethylene oxide and 125 mL of isopropanol were added to a 1-L beaker, and while stirring at 300 to 400 rpm using an impeller, 588 g of ion-exchange water was added, and the mixture was stirred for 1 minute. Then, the stirring speed was changed to 60 rpm, and stirring was further continued for 3 hours, thereby obtaining a 2% aqueous solution of polyethylene oxide. The aqueous solution was maintained at 25°C, the viscosity was measured using a rotational viscometer (RV DVII+, produced by Brookfield), and this value was taken as the 2% aqueous solution viscosity.

Gel Strength

200 mg of polyethylene oxide was placed in a cylindrical mortar with a diameter of 10 mm and compressed with a compression force of 5 kN to obtain tablets. Using the tablets, a dissolution test was performed according to the Japanese Pharmacopoeia (paddle method). In the dissolution test, the test liquid was ion-exchange water, the test temperature was 37°C, and the stirring speed was 200 rpm. The dissolution test was performed by stirring under these conditions for 2.5 hours to gelate the tablets. Using the gelled tablets as measurement samples, a compression test was performed using a compact compression/tensile tester (EZ-Test, produced by Shimadzu Corporation). In the compression test, an indenter with a diameter of 10 mm was used, and the displacement rate was 5 mm/min. Based on a stress-strain curve obtained by the compression test, the slope S (Pa) when the gelled tablets were displaced from 5% to 10% was derived. The above series of operations were performed three times in total with different tablets, and the slope S (Pa) of each case was derived. The average value of these was taken as the gel strength of the polyalkylene oxide.

Erosion; Dissolution Rate

200 mg of polyalkylene oxide was placed in a cylindrical mortar with a diameter of 10 mm and compressed with a compression force of 5 kN to obtain tablets. Using the tablets, a dissolution test was performed according to the Japanese Pharmacopoeia (paddle method). In the dissolution test, the test liquid was ion-exchange water, the test temperature was 37°C, and the stirring speed was 200 rpm. The dissolution test was performed by stirring under these conditions for 4.5 hours to gelate the tablets. The gelled tablets were taken out and dried at 80°C for 5 hours, and the erosion (dissolution rate) was calculated from the following formula (1):

$$\text{Erosion (\%)} = \{1-(A/B)\} \times 100 \quad (1)$$

(In formula (1), A represents tablet mass after drying performed after the dissolution test, and B represents tablet mass before the dissolution test.)

**Claims**

1. A polyalkylene oxide having a polydispersity of 3 to 5, a gel strength of 22500 Pa or more, and an erosion of 40 to 48%.

2. A solid dosage form composition comprising at least the polyalkylene oxide according to claim 1, an active component, and an excipient.

3. A sustained-release dosage form composition comprising at least the polyalkylene oxide according to claim 1, an active component, and an excipient.

4. A method for producing a solid dosage form composition, comprising:

   dry-mixing the polyalkylene oxide according to claim 1 and an active component to obtain a mixture; and tableting the mixture to obtain tablets.

5. A method for producing a solid dosage form composition, comprising:

   obtaining granules comprising the polyalkylene oxide according to claim 1 and an active component; and tableting the granules to obtain tablets.

# EP 4 174 109 A1

## INTERNATIONAL SEARCH REPORT

| International application No. |
|---|
| **PCT/JP2022/027315** |

**A. CLASSIFICATION OF SUBJECT MATTER**

*C08G 65/06*(2006.01)i; *C08G 65/30*(2006.01)i; *A61K 9/22*(2006.01)i; *A61K 47/34*(2017.01)i
FI:  C08G65/06; A61K9/22; C08G65/30; A61K47/34

According to International Patent Classification (IPC) or to both national classification and IPC

**B. FIELDS SEARCHED**

Minimum documentation searched (classification system followed by classification symbols)

C08G65/06; C08G65/30; A61K9/22; A61K47/34

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 5-178981 A (BAYER AKTIENGESELLSCHAFT) 20 July 1993 (1993-07-20) claims 1, 2, paragraphs [0016], [0024], examples 4-6 | 1-5 |
| Y | | 2-5 |
| X | JP 11-12351 A (MITSUI CHEMICALS INC.) 19 January 1999 (1999-01-19) example 2 | 1 |
| Y | | 2-5 |
| X | JP 2001-514280 A (ARCO CHEMICAL TECHNOLOGY LP   ) 11 September 2001 (2001-09-11) claim 10, examples 8, 9 | 1 |
| Y | | 2-5 |
| Y | WO 2013/080888 A1 (SUMITOMO SEIKA CHEMICALS CO., LTD.) 06 June 2013 (2013-06-06) claim 1, paragraph [0001] | 2-5 |
| A | | 1 |

☑ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 September 2022** | **20 September 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**EP 4 174 109 A1**

**INTERNATIONAL SEARCH REPORT**

| International application No. |
| --- |
| **PCT/JP2022/027315** |

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2014/191397 A1 (GRUENENTHAL GMBH) 04 December 2014 (2014-12-04) claims 1, 7, 9, 11, 22, p. 61, paragraph 7 | 2-5 |
| A | | 1 |
| A | JP 2005-281665 A (ASAHI KASEI CORP.) 13 October 2005 (2005-10-13) | 1-5 |

**INTERNATIONAL SEARCH REPORT**
**Information on patent family members**

International application No.

**PCT/JP2022/027315**

| Patent document cited in search report | | | Publication date (day/month/year) | Patent family member(s) | | | Publication date (day/month/year) |
|---|---|---|---|---|---|---|---|
| JP | 5-178981 | A | 20 July 1993 | EP | 515949 | A2 | |
| | | | | claims 1, 9, paragraph [0024], examples 4-6 | | | |
| JP | 11-12351 | A | 19 January 1999 | (Family: none) | | | |
| JP | 2001-514280 | A | 11 September 2001 | WO | 1999/010407 | A1 | |
| | | | | claim 10, examples 8, 9 | | | |
| | | | | EP | 1012203 | A1 | |
| | | | | CN | 1310735 | A | |
| WO | 2013/080888 | A1 | 06 June 2013 | US | 2015/0141705 | A1 | |
| | | | | claim 1, paragraph [0080] | | | |
| | | | | EP | 2787023 | A1 | |
| | | | | CN | 103946269 | A | |
| WO | 2014/191397 | A1 | 04 December 2014 | US | 2014/0356426 | A1 | |
| | | | | EP | 3003279 | A1 | |
| JP | 2005-281665 | A | 13 October 2005 | (Family: none) | | | |

Form PCT/ISA/210 (patent family annex) (January 2015)

**REFERENCES CITED IN THE DESCRIPTION**

**Patent documents cited in the description**

- JP 2014009208 A